# EUROPÄISCHE PATENTSCHRIFT

(11) **EP 1 923 037 B1**
(45) Veröffentlichungstag und Bekanntmachung des Hinweises auf die Patenterteilung: **09.03.2011**
(21) Anmeldenummer: 07021672.6
(22) Anmeldetag: 08.11.2007
(51) Int. Cl.: A61K 6/083

(54) **Polymerisierbares Dentalmaterial auf der Basis von Methylmethacrylaten, Geeignet zur Herstellung von Prothesenkunststoff**
Polymerisable dental material based on methyl methacrylates, suitable for manufacturing prosthetic synthetic material
Matériau dentaire polymérisable à base de méthylméthacrylates adapté à la fabrication de matière synthétique de prothèse

(30) Priorität: 20.11.2006 DE 102006054879
(43) Veröffentlichungstag der Anmeldung: 21.05.2008
(73) Patentinhaber: Heraeus Kulzer GmbH, 63450 Hanau (DE)
(72) Erfinder: Kerscher, Kevin, 61381 Friedrichsdorf (DE); Savic, Novica, 63526 Erlensee (DE); Renz, Karl-Heinz, 60489 Frankfurt (DE)
(74) Vertreter: Kühn, Hans-Christian

(56) Entgegenhaltungen:
- EP-A- 0 584 376
- EP-A- 1 702 633
- EP-A2- 0 411 512
- WO-A-86/05793
- DE-C- 940 493
- US-A- 3 427 274

## Beschreibung

Die Erfindung betrifft polymerisierbares Dentalmaterial auf der Basis von Methylmethacrylaten, geeignet zur Herstellung von Prothesenkunststoff.

### Technischer Hintergrund

Zur Herstellung von Totalprothesen, Teilprothesen, Zahnspangen etc. für das Tragen im Mund sind verschiedene Materialien erhältlich:
1. Heißpolymerisierende Kunststoffe (1 oder 2 Komponenten). Diese weisen einen sehr hohen thermisch bedingten Volumenschrumpf auf, der zu Passungenauigkeiten führt.
2. Autopolymerisierende Kunststoffe (2 Komponenten).
3. Lichtpolymerisierende Kunststoffe (1 oder 2 Komponenten).
4. Thermoplastische Kunststoffe (1 Komponente), solche Materialien sind zahntechnisch relativ schwer zu verarbeiten.
5. Mikrowellenhärtende Kunststoffe (1 oder 2 Komponenten). Auch hier führt ein sehr hoher thermisch bedingter Volumenschrumpf zu Passungenauigkeiten

Aus den unter 1. bis 5. genannten Materialien gefertigte Prothesen können beim Herunterfallen oder sonstiger unachtsamer Behandlung leicht brechen. Man ist daher seit längerem bestrebt, die Kunststoffe für zahntechnische Prothesen bruchfest auszurüsten. Die unerwünschte Bruchneigung wird etwa durch Verwendung sogenannter High Impact Kunststoffe beseitigt¹
¹ (Der Begriff High-lmpact wird in der ISO 1567 - Denture Base Materials - näher erläutert. Danach ist der Prothesenkunststoff dann ein High-lmpact-Denture-Base-Material, wenn er in der Schlagzähigkeit nach ISO 1567 (an Charpy angelehnt) einen Wert von 2 kJ/m² übersteigt.):

6. Heißpolymerisierende High-Impact-Kunststoffe (1 oder 2 Komponenten). Diese weisen allerdings wiederum den unerwünscht hohen thermisch bedingten Volumenschrumpf auf, der zu Passungenauigkeiten führt

Es sind bereits Prothesenkunststoffe auf den Markt gekommen, welche das Problem des Schrumpfens durch Zugabe von Butadien-Styrol-Kautschuk gelöst haben (EP 1 702 633 A2).

Nachteil dieser Technologie ist, dass die so erhaltenen Kunststoffe immer eine Trübung mit sich bringen. Selbst wenn die Teilchengröße des Kautschuks kleiner als der sichtbare Wellenlängenbereich des Lichts gewählt wird, bleibt dennoch ein opaleszierender Eindruck.

In einem Artikel von Kerby et al. -- "Fracture toughness of modified dental resins" [J. of Oral Rehabilitation 30, 780-4 (2003)] -- wird die Verwendung von methacrylatterminiertem 1,3-Butadien-Acrylnitril-Acrylsäure-Terpolymer in Dentalmaterial, vornehmlich auf Basis von TEGDMA (Triethylenglycoldimethacrylat) beschrieben. Das Terpolymer ist unter dem Handelsnamen Hycar Reactive Liquid Polymer 1300 x 33 erhältlich. Die Publikation geht nicht auf die optischen Eigenschaften ein.

Gemäß DE 196 17 876 A1 werden Polysiloxanpfropfcopolymerisaten als Schlagzähmodifikatoren eingesetzt: ein Polysiloxan-Pfropfcopolymerisat, welches einen Kern aus elastomerem Polysiloxan und eine Hülle aus nicht-elastomerem Polymer aufweist, und/oder (2) ein Polysiloxan mit (Meth)acryl-Gruppen.

US 5,182,332 beschreibt den Einsatz von (Meth)acrylat-gepfropften Butadienkautschuken als Zusatz zu Prothesenbasismaterial. Es handelt sich um die bereits erwähnten marktüblichen High-Impact-Prothesenkunststoffe. Mit den dort beschriebenen Materialien sind allerdings keine transparenten Werkstoffe realisierbar. In US 5,182,332 werden außerdem vorzugsweise feste Mehrschichtkautschuke eingesetzt (vgl. Anspruch 1).

Zweikomponentige Pulver-Flüssigkeits-Systeme bestehen in der Regel hauptsächlich aus Meth- acrylaten. Die Methacrylate sind als Pulver und als Flüssigkeiten erhältlich. Das Polymerpulver ist heutzutage meist ein Perlpolymerisat. Es wird mit flüssigem Monomer im Gewichtsverhältnis von 2,5-3 : 1 vermischt. Nach einer Anschwellzeit ergibt sich ein Teig, der gepresst, gegossen oder geformt werden kann. Typische derartige Zusammensetzungen sind z.B. in DE 737 058 A und DE 37 25 502 A1 beschrieben.

Es stellt sich die Aufgabe, polymerisierbares Dentalmaterial auf der Basis von Methylmethacrylaten, geeignet zur Herstellung von Prothesenkunststoff, bereit zu stellen, mit dem die oben beschriebenen Nachteile ganz oder teilweise vermieden werden oder dessen Eigenschaften verbessert sind.

### Erfindung

Überraschenderweise hat sich herausgestellt, dass acrylierte oder methacrylierte Butadien- und/oder Acrylnitril-Oligomere oder Polymere auch in auf Methylmethacrylat (MMA) basierenden Systemen vorteilhafte Eigenschaften bewirkt, wenn sie als flüssige Polymere (Oligomere) zugesetzt werden. Es ergeben sich transparente Produkte, die nach dem Aushärten eine Transparenz von > 70 % bei einer Schichtdicke von 3 mm aufweisen. Weiterhin ist von Vorteil, dass nach Härtung Bruchzähigkeit, Brucharbeit und Schlagzähigkeit bereits ab einem Anteil von ca. 1% steigen - bei gleichzeitig gleichbleibenden Biegefestigkeits- und Biegemodulwerten.

Daraus ergibt sich eine erhöhte Bruchfestigkeit während der Lebensdauer/Anwendung des
polymerisierten Dentalmaterials.
Als Modifizierungsmittel können insbesondere verwendet werden:
- methacryliertes Butadien Oligomer,
- acryliertes Butadien Oligomer,
- methacryliertes Acrylnitril-Butadien Oligomer,
- acryliertes Acrylnitril-Butadien Oligomer,
- weitere acrylierten/methacrylierten Oligomere/Polymere enthaltend Butadien und/oder Acrylnitril.

Besonders bevorzugt ist ein methacryliertes Acrylnitril-Butadien-Oligomer zu verwenden.

Die Erfindung betrifft somit ein polymerisierbares Dentalmaterial aufweisend: eine Flüssigkomponente enthaltend
A) mindestens eine flüssige Methylmethacrylat-Monomerkomponente,
B) mindestens
   - ein acryliertes oder methacryliertes Butadien-Oligomer oder -Polymer und/oder
   - ein acryliertes oder methacryliertes Acrylnitril-Butadien-Oligomer oder Polymer,
sowie eine Pulverkomponente enthaltend ein Polymerpulver oder ein Perlpolymerisat.

Das Material besitzt nach dem Aushärten eine Lichtdurchlässigkeit (Transparenz) im sichtbaren Bereich von > 70 % bei einer Schichtdicke von 3 mm.

Zusätzlich können ein oder mehrere Stoffe aus den Gruppen der weiteren Monomeren, Füllstoffe, Pigmente, Stabilisatoren, Regler, antimikrobiellen Additive, UV-Absorber, Thixotropiermittel, Katalysatoren und Vernetzer enthalten sein.

Als weitere Monomere kommen die auf dem Dentalgebiet üblichen Monomere in Betracht:
Beispiele sind radikalisch polymerisierbare monofunktionelle Monomere wie Mono(meth)-acrylate, Methyl-, Ethyl-, Butyl-, Benzyl-, Furfuryl- oder Phenyl(meth)acrylat, di- bzw. polyfunktionelle Monomere wie di- oder polyfunktionelle Acrylate bzw. Methacrylate, z.B. Bisphenol-A-di(meth)acrylat, Bis-GMA (ein Additionsprodukt aus Methacrylsäure und Bisphenol-A-diglycidyl-ether), UDMA (ein Additionsprodukt aus 2-Hydroxyethylmethacrylat und 2,2,4-Hexamethylendiisocyanat), Di-, Tri- oder Tetraethylenglykoldi(meth)acrylat, Decandioldi(meth)acrylat, Dodecandioldi(meth)acrylat, Hexyldecandioldi(meth)acrylat, Trimethylolpropantri(meth)acrylat, Pentaerythrittetra(meth)-acrylat sowie Butandioldi(meth)acrylat, Ethylenglycol-di(meth)acrylat, Polyethylenglycol-di(meth)acrylate, ethoxylierte/propoxylierte Bisphenol-A-di(meth)acrylate.

Als Füllstoffe kommen z.B. pyrogene oder Fällungskieselsäuren, Dentalgläser wie Aluminosilicatgläser oder Fluoroaluminosilicatgläser, Strontiumsilicat, Strontiumborosilicat, Lithiumsilicat, Lithiumaluminiumsilicat, Schichtsilikate, Zeolithe, amorphe sphärische Füller auf Oxid- oder Mischoxidbasis (SiO₂, ZrO₂ und/oder TiO₂), Metalloxide mit Primärteilchengröße von ca. 40 bis 300 nm, Splitterpolymerisate mit 10- 100 µm Teilchengröße (vgl. R. Janda, Kunststoffverbundsysteme, VCH Verlagsgesellschaft, Weinheim, 1990, Seite 225 ff.) oder deren Mischungen in Frage. Zudem können Verstärkungsmittel wie Glasfasern, Polyamid- oder Kohlenstoffasern eingearbeitet werden.

Die Füllstoffe werden in der Regel zu 0 bis 80 Gew.%, vorzugsweise zu 0 bis 3 Gew. %, bezogen auf die gesamte Prothesenkunststoffzusammensetzung bzw. die Summe der Komponenten A und B, eingesetzt.

Als Regler zur Einstellung des Molekulargewichts kommen z.B. in Frage:
TGEH: Thioglykolsäure-2-ethylhexylester,
t-DDM: tert.-Dodecylmercaptan,
GDMA: Glykoldimercaptoacetat.

Beispiele für Initiatoren sind:
LPO: Dilauroylperoxid,
BPO: Dibenzoylperoxid,
t-BPEH: tert.-Butylper-2-ethylhexanoat,
ADMV: 2,2'-Azobis(2,4-dimethylvaleronitril),
AIBN: 2,2'-Azobis-(isobutyronitril),
DTBP: Di-tert.-butylperoxid.

Geeignete Stabilisatoren sind z. B. Hydrochinonmonomethylether oder 2,6-Di-tert.-butyl-4-methylphenol (BHT).

Des weiteren können die erfindungsgemäßen Prothesenbasismaterialien weitere übliche Zusatzstoffe enthalten, z.B. aus den Gruppen der antimikrobiellen Additive, UV-Absorber, Thixotropiermittel, Katalysatoren und Vernetzer.

Solche Additive werden - wie die Pigmente, Stabilisatoren und Regler - in eher geringen Mengen eingesetzt, insgesamt 0,01 bis 3,0, besonders 0,01 bis 1,0 Gew.% bezogen auf die Gesamtmasse des Materials.

Die Aushärtung der Zusammensetzungen erfolgt vorzugsweise durch redoxinduzierte radikalische Polymerisation bei Raumtemperatur bzw. bei leicht erhöhter Temperatur unter leichtem Druck, um Blasenbildung zu vermeiden.
Als Initiatoren für die bei Raumtemperatur durchgeführte Polymerisation werden z.B. Redoxinitiator-Kombinationen, wie z.B. Kombinationen von Benzoyl- oder Laurylperoxid mit N,N-Dimethyl-sym.-xylidin oder N,N-Dimethyl-p-toluidin, verwendet. Ein besonders bevorzugtes
Initiatorsystem ist eine Kombination von Barbitursäuren in Verbindung mit Kupfer- und Chlorid-ionen sowie oben genannten Peroxiden. Dieses System zeichnet sich durch eine hohe Farbstabilität aus.

Die Materialien der Erfindung werden bevorzugt im Dentalbereich angewandt, vor allem zur Herstellung von Prothesen oder kieferorthopädische Apparaturen zur Korrektur der Zahnstellung.

Der Anteil an Komponente in den erfindungsgemäßen Zusammensetzungen A) beträgt vorzugsweise über 20 Gew %, bevorzugt >20 bis 99 Gew. %, besonders >20 bis 98 Gew. %, ganz besonders 20 bis 50 Gew. %.

Der Anteil an Komponente B) beträgt vorzugsweise über 1 Gew. %, weiter bevorzugt bis 99%, insbesondere 2-80 Gew.%, ganz besonders 50 -80 Gew.%.

Die erfindungsgemäßen Materialien eignen sich besonders zur Anwendung im Dentalbereich bei der Herstellung von Prothesen. Weitere Möglichkeiten bestehen in allen Bereichen, in denen ein bruchfester Formkörper individuell erstellt werden muss, z.B. bei
- Knochenzementen mit verbesserter Schlagzähigkeit,
- veterinärmedizinischen Anwendungen, bei denen die Schlagzähigkeit hoch sein muss, z.B. Hufreparaturmaterial oder Zahnersatz für Tiere,
- kieferorthopädischen Apparaturen zur Korrektur der Zahnstellung.
Die folgenden Beispiele erläutern die Erfindung näher, ohne sie zu beschränken. Teile- und Prozentangaben beziehen sich wie in der übrigen Beschreibung auf das Gewicht, sofern nicht anders angegeben:

### Beispiel:

Die folgende Versuchstabelle zeigt, daß sich vor allem der Elastizitätsmodul sich bei erfindungsgemäßer Zugabe der Copolymeren sehr vorteilhaft verändert. Versuchsgrundlage war herkömmliches, mit Dibenzoylperoxid kaltpolymerisierendes² Prothesenmaterial PalaXpress (Pulver/Flüssigkeit jeweils auf Methacrylatbasis), wobei der Modifier mit der Flüssigkeit homogenisiert wurde.
² **Kaltpolymerisate (auch** "**Warmpolimerisate**") (Handelsnamen: *Trigon 40, PalaXpress, Castodon it)* weisen ein spezielles Katalysatorsystem auf, welches die Polymerisation zwar auslöst, aber gleichzeitig so weit verzögert, dass eine ausreichend lange Verarbeitungszeit gewährleistet ist. Diese Kunststoffe können daher, bevor sie anquellen, auch gegossen werden und haben damit eine universelle Verarbeitungsbreite (Gießtechnik, Injektionstechnik, Totalprothetik, partielle Prothetik).

Die Untersuchungsergebnisse der polymerisierten Proben sind wie folgt:

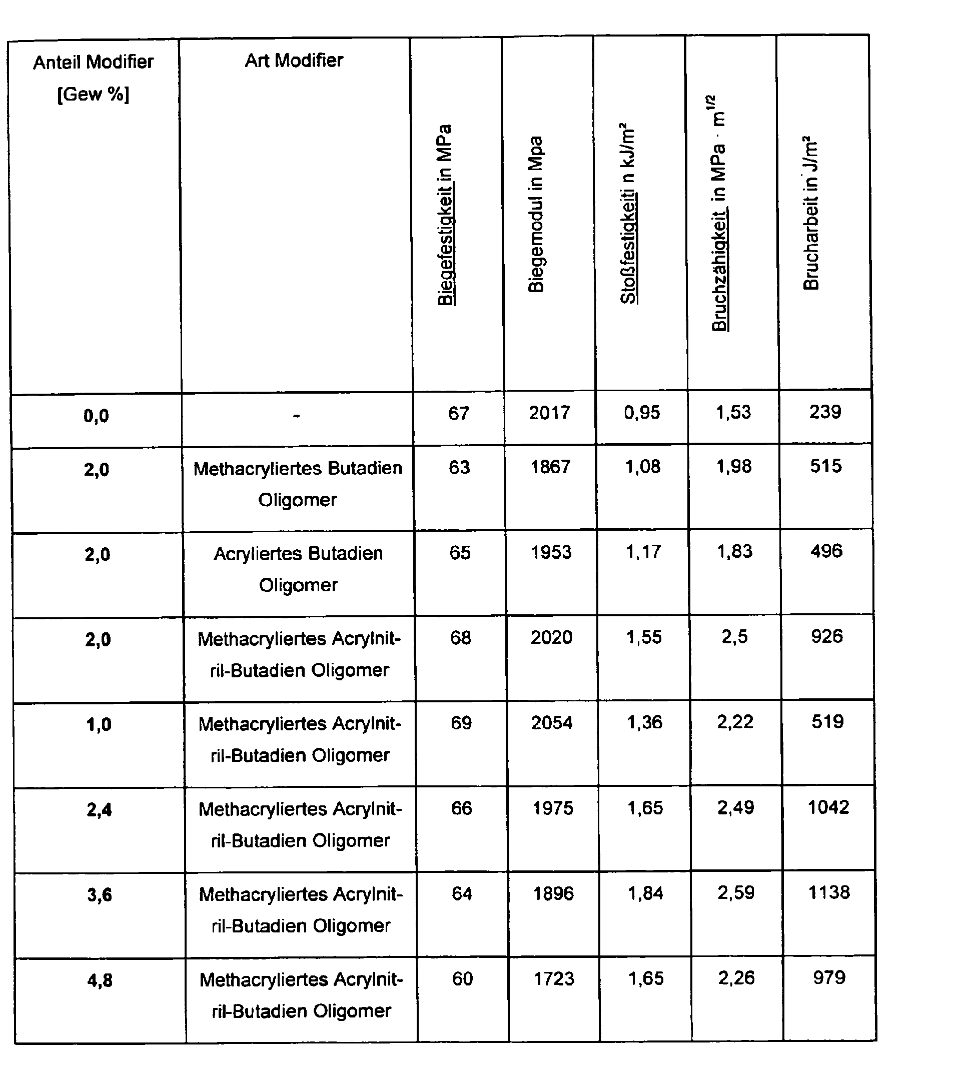

## Patentansprüche

1. Polymerisierbares Dentalmaterial aufweisend die Merkmale I-III, nämlich
I eine Flüssigkomponente enthaltend
A) mindestens eine flüssige Methylmethacrylat-Monomerkomponente,
B) mindestens ein als flüssiges Polymere (Oligomer) zugesetztes ein acryliertes oder methacryliertes Butadien-Oligomer oder -Polymer und/oder
• ein acryliertes oder methacryliertes Acrylnitril-Butadien-Oligomer oder -Polymer,
II eine Pulverkomponente enthaltend ein Polymerpulver oder ein Perlpolymerisat auf Methacrylat-Basis,
III nach dem Aushärten eine Transparenz von > 70 % bei einer Schichtdicke von 3 mm,
wobei der Anteil an Komponente B) > 1 Gew % bezogen auf das Dentalmaterial beträgt, und der Anteil an Komponente A) >20 Gew. %.

2. Dentalmaterial nach Anspruch 1, zusätzlich enthaltend einen oder mehrere Stoff(e) aus den Gruppen der weiteren Monomeren, Füllstoffe, Pigmente, Stabilisatoren, Regler, antimikrobiellen Additive, UV-Absorber, Thixotropiermittel, Katalysatoren und Vernetzer.

3. Dentalmaterial nach einem der vorstehenden Ansprüche mit Anteil an Komponente B) von > 1 bis 99 Gew %.

4. Dentalmaterial nach einem der vorstehenden Ansprüche mit Anteil an Komponente B) von 2 bis 80 Gew%.

5. Dentalmaterial nach einem der vorstehenden Ansprüche mit Anteil an Komponente B) von 50 bis 80 Gew %.

6. Dentalmaterial nach einem der vorstehenden Ansprüche mit Anteil an Komponente A) von > 20 bis 99 Gew. %.

7. Dentalmaterial nach einem der vorstehenden Ansprüche mit Anteil an Komponente A) >20 bis 50 Gew. %.

8. Verwendung von Dentalmaterial nach einem der vorstehenden Ansprüche zur Bereitstellung von
a. Knochenzement mit verbesserter Schlagzähigkeit,
b. Hufreparaturmaterial oder Zahnersatz für Tiere,
c. kieferorthopädischen Apparaturen zur Korrektur der Zahnstellung.

## Claims

1. Polymerisable dental material comprising features I-III, namely
I a liquid component containing
A) at least one liquid methyl methacrylate monomer component;
B) at least
• one acrylated or methacrylated butadiene oligomer or polymer and/or
• an acrylated or methacrylated acrylonitrile-butadiene oligomer or polymer, added as liquid polymer (oligomer);
II a powder component containing a polymer powder or a bead polymer based on methacrylate,
III, after curing, a transparency of > 70 % at a layer thickness of 3 mm;
whereby the proportion of component B) > 1 % by wt. relative to the dental material and the proportion of component A) > 20 % by wt.

2. Dental material according to claim 1, additionally containing one or more substance(s) from the group of further monomers, fillers, pigments, stabilisers, regulators, antimicrobial additives, UV absorbers, thixotroping agents, catalysts and crosslinking agents.

3. Dental material according to any one of the preceding claims with a proportion of component B) of > 1 to 99 % by wt.

4. Dental material according to any one of the preceding claims with a proportion of component B) of 2 to 80 % by wt.

5. Dental material according to any one of the preceding claims with a proportion of component B) of 50 to 80 % by wt.

6. Dental material according to any one of the preceding claims with a proportion of component A) of > 20 to 99 % by wt.

7. Dental material according to any one of the preceding claims with a proportion of component A) of >20 to 50 % by wt.

8. Use of dental material according to any one of the preceding claims to provide
a. bone cement with improved impact resistance,
b. hoof repair material or dental prosthesis for animals,
c. orthodontic devices for correcting the position of teeth.

## Revendications

1. Matériau dentaire polymérisable présentant les caractéristiques I à III, à savoir
I un composant liquide contenant
A) au moins un composant monomère de méthacrylate de méthyle liquide,
B) au moins un (oligomère) en tant que polymères liquides ajouté
• un oligomère ou polymère de butadiène acrylé ou méthacrylé et/ou
• un oligomère ou polymère d'acrylonitrile butadiène acrylé ou méthacrylé,
II un composant pulvérulent contenant une poudre de polymère ou un polymérisat nacré à base de méthacrylate,
III après le durcissement, une transparence de > 70 % avec une épaisseur de couche de 3 mm,
dans lequel la proportion en composant B) se monte à > 1 % en poids par rapport au matériau dentaire, et la proportion en composant A) à > 20 % en poids.

2. Matériau dentaire selon la revendication 1, contenant en outre une ou plusieurs substance(s) parmi les groupes des autres monomères, charges, pigments, stabilisants, régulateurs, additifs anti-microbiens, absorbeurs d'UV, agents thixotropes, catalyseurs et agents de réticulation.

3. Matériau dentaire selon l'une quelconque des revendications précédentes avec une proportion en composant B) de > 1 à 99 % en poids.

4. Matériau dentaire selon l'une quelconque des revendications précédentes avec une proportion en composant B) de 2 à 80 % en poids.

5. Matériau dentaire selon l'une quelconque des revendications précédentes avec une proportion en composant B) de 50 à 80 % en poids.

6. Matériau dentaire selon l'une quelconque des revendications précédentes avec une proportion en composant A) de > 20 à 99 % en poids.

7. Matériau dentaire selon l'une quelconque des revendications précédentes avec une proportion en composant A) de > 20 à 50 % en poids.

8. Utilisation de matériau dentaire selon l'une quelconque des revendications précédentes pour la préparation de
a. ciment osseux avec une résilience améliorée,
b. matériau de réparation de sabot ou de remplacement dentaire pour animaux,
c. appareils d'orthopédie maxillaire pour la correction de la position dentaire.
